(19) **Europäisches Patentamt**

**European Patent Office**

**Office européen des brevets**

(11) **EP 0 971 686 B1**

(12) **EUROPEAN PATENT SPECIFICATION**

(45) Date of publication and mention
of the grant of the patent:
**03.11.2004 Bulletin 2004/45**

(51) Int Cl.⁷: $A61K\ 7/48$

(86) International application number:
**PCT/EP1998/002115**

(21) Application number: **98925469.3**

(22) Date of filing: **31.03.1998**

(87) International publication number:
**WO 1998/044904 (15.10.1998 Gazette 1998/41)**

(54) **COSMETIC AND/OR DERMATOLOGICAL COMPOSITION CONTAINING A DERIVATIVE OF METHYLATED SILANOL AND A DERIVATIVE OF HYDROLYSED PLANT PROTEIN**

KOSMETISCHE UND/ODER DERMATOLOGISCHE ZUSAMMENSETZUNG DIE EIN METHYLIERTES SILANOLDERIVAT UND EIN HYDROLISIERTES PFLANZLICHES EIWEISS ENTHÄLT

PREPARATION COSMETIQUE ET/OU DERMATOLOGIQUE CONTENANT UN DERIVE DE SILANOL METHYLE ET UN DERIVE DE PROTEINE VEGETALE HYDROLYSEE

(84) Designated Contracting States:
**AT BE CH DE DK ES FI FR GB GR IE IT LI LU MC NL PT SE**
Designated Extension States:
**LT LV RO SI**

(30) Priority: **04.04.1997 FR 9704167**

(43) Date of publication of application:
**19.01.2000 Bulletin 2000/03**

(73) Proprietor: **Beiersdorf AG**
**20253 Hamburg (DE)**

(72) Inventors:
• **FRUCTUS, Alain, E.**
  **F-92400 Courbevoie (FR)**
• **MONTET, Florence**
  **F-92300 Levallois Perret (FR)**
• **LAZAR, Gabriela**
  **F-75017 Paris (FR)**
• **TOKGOZ, Nur, Selcan**
  **F-75013 Paris (FR)**

(56) References cited:
CH-A- 686 997          DE-A- 3 938 284
FR-A- 2 499 405        FR-A- 2 561 915
FR-A- 2 597 337        FR-A- 2 667 240
US-A- 4 382 961

• "Use of Silanols in Cosmetics Promoted at Tri-K Seminar" SOAP, COSMETICS AND CHEMICAL SPECIALTIES, vol. 65, no. 5, 1989, page 80 XP000046891

EP 0 971 686 B1

**Description**

This invention relates to the treatment of the skin for example to treat the symptoms of skin ageing by preventing irreversible cross-links of the proteins of the connective tissue and to minimise the effects of atmospheric pollution.

Ageing is a natural process which results from the progressive decline of the function of an organism. During ageing, extensive modifications occur in each organ, particularly in the connective tissue.

For example, collagen, the most abundant protein in the human body, becomes more insoluble, more resistant to digestion, to thermal rupture and to mechanical tension. In the case of skin ageing, these modifications of the physicochemical properties of collagen contribute to the development of long-term complications, such as loss of elasticity, suppleness and tonicity

Collagen is a fibrous protein composed of three polypeptide chains (tropocollagen fibrils) coiled in a triple helix. These polypeptide chains are of equal length and each has about one thousand amino acid groups. They mainly contain 35 percent glycine, 21 percent proline, 12 percent hydroxyproline and 11 percent alanine residues.

X-ray diffraction analyses have shown that each polypeptide chain of tropocollagen itself forms a triple helix. They also present cross linkages between them formed by hydrogen bridges and an unusual type of covalent cross-link, which is only found in collagen (formed between the lysine residues of two chains). Tropocollagen also contains carbohydrate side chains linked to the hydroxyl groups of the hydroxylysine.

The synthesis of collagen molecules inside the cell is a complex process and requires major intracellular and extracellular post-translational modifications. In the intracellular space, during biosynthesis, some residues of lysine and proline are hydroxylated, and these hydroxylated residues are glycosylated by an enzyme. 'Glycosylation' means the bonding of a sugar having six carbon atoms with the free amino group of a protein. This bonding is also known in the art by the name of glucosylation or glycation. These hydroxylation and glycosylation reactions of tropocollagen are necessary for it to be secreted outside the cell. Once secreted in the extracellular space, the tropocollagen chains are linked by covalent bonds and form fibrillary networks with cross linkages.

In addition to the enzymatic glycosylation of the amino acid groups of tropocollagen, the non-enzymatic glycosylation of certain residues of lysine and hydroxylysine also occurs in the extracellular space. In fact, the non-enzymatic addition of any sugar having six carbon atoms to the free amino acid groups of the protein causes structural and functional modifications of the tissue. In the case of collagen, this leads to the formation of irreversible cross links between the collagen fibres, which ultimately results in a stiffening of the tissue and a loss of elasticity of the skin (Cerami et al 1987).

The non-enzymatic glycosylation of the proteins and its chemical consequences have been known for a long time, and by reference to the so-called Maillard or Browning reactions of sugars in food chemistry. These reactions cause the condensation of a glycoaldehyde or a ketone with a free amino group of a protein, producing a glycosylamine (Schiffs base). The resulting product may undergo an Amadori rearrangement to form the more stable Amadori product. This product can then initiate a series of dehydrations and rearrangements to form highly reactive carbonyl compounds identified by their fluophoric and chromophoric properties. The fluophores and chromophores resulting from these chain reactions have been designated by the names of end product of evolved glycosylation (EPEG) of evolved Browning products or Maillard products (in food chemistry). The reactive carbonyl groups of EPEG are capable of forming irreversible cross linkages with other amino groups of the protein, giving rise to a decrease in solubility of the protein, which is one of the causes of the ageing process (Cerami et al 1987, Brownlee et al 1986, Shin et al 1988).

The prevention of the formation of irreversible cross linkages between the collagen fibres by non-enzymatic glycosylation is a concern of all 'anti-ageing' treatments. One of the methods used consists in inactivating the glycosylation products at an early stage (Schiffs base and addition products resulting from an Amadori rearrangement) by blocking their reactive carbonyl groups. In this field, some investigations have been conducted with a nucleophilic hydrazine compound, aminoguanidine (Cerami et al 1987, Brownlee et al 1986) and its derivative, guanobenzoacetate (Igaki et al 1991). Another approach aimed to prevent the formation of cross linkages between the proteins in connection with anti-ageing treatment consists in removing the EPEG products by activation of macrophages (Cerami et al 1987).

In everyday life the skin is exposed to atmospheric pollution in the form of, for example, the emissions from motor vehicles or from tobacco smoke. These emissions can cause a reduction in the moisture of the skin and can lead to undesirable dermatological effects. There is therefore a need for dermatological and cosmetic compositions which prevent the adverse consequences of exposure to atmospheric pollution. The present invention aims to provide such dermatological and cosmetic compositions.

Document CH-A-686997 describes the use oft a combination of monoethylsilanol, polypetides derived from elastin, plant ceramides and extracts of algae in cosmetics. Monomethycylanol and peptides derived from elastin are mentioned in concentrations of 30 to 51 wt.%.

The document FR 2561915 describes the use of derivatives of Silanol, e.g. Trimethylsilanol in combination with water soluble polymers, e.g. collagen, hyaluronic acid as skin moisturizer.

This invention mainly relates to the combination of derivatives of methylated silanols with derivatives of hydrolysed plant protein to prevent the consequences of the symptoms of ageing of the skin by avoiding irreversible cross-links of the proteins of the connective tissue and to prevent the consequences of exposure to atmospheric pollution.

The invention hence relates to a dermatological and cosmetic composition for treating symptoms of skin ageing and to prevent the consequences of exposure to atmospheric pollution comprising a combination of at least one derivative of methylated silanol and at least one derivative of hydrolysed plant protein.

It is a further aim of this invention to use the combination of derivatives of methylated silanols, extract of hydrolysed plant protein, and vitamin C (and/or its derivatives, particularly magnesium ascorbyl phosphate to prevent the consequences of symptoms of skin ageing by stimulating the synthesis of new collagen and by maintaining the degree of glycosylation on the newly synthesized collagen at a constant value and to prevent the consequences of exposure to atmospheric pollution.

The invention hence also relates to a dermatological and cosmetic composition as aforedescribed and which furthermore contains vitamin C and/or one or a plurality of its derivatives.

Compositions of the present invention which additionally contain oils extracted from vegetable sources such as Helianthus annuus and Hedera helix and/or phytic acid for example extracted from the bran of rice have particular utility in the treatment of the consequences of exposure to atmospheric pollution.

The present invention therefore also relates to a dermatological and cosmetic composition as aforedescribed and which furthermore contains oils extracted from vegetable sources such as Helianthus annuus and Hedera helix and/or phytic acid for example extracted from the bran of rice.

Compositions of the present invention which additionally contain at least one oligopeptide or a derivative thereof provide additional antiglycation properties. Suitable oligopeptides or derivatives thereof include palmitoyl oligopeptide in which the oligopeptide is composed of glycine histidine and lysine moieties or arginine, glycine aspartic acid and serine moieties.

The present invention therefore also relates to a dermatological and cosmetic composition as aforedescribed which furthermore contains an oligopeptide or a derivative thereof.

It is a further object of this invention to use the combination of derivatives of methylated silanols, extract of hydrolysed plant proteins (particularly extract of hydrolysed wheat proteins), vitamin C (and/or its derivatives, particularly magnesium ascorbyl phosphate), and vitamin E (and/or skin by inhibiting the production of free radicals.

Hence the invention also relates to a dermatological and cosmetic composition comprising one or a plurality of derivatives of methylated silanol, an extract of hydrolysed plant proteins (particularly an extract of hydrolysed wheat protein). vitamin C (and/or its derivatives, particularly magnesium ascorbyl phosphate), and vitamin E (and/or its derivatives).

In particular, the compositions of this invention, which are judged useful for the treatment of symptoms of ageing and for the prevention of the consequences of exposure to atmospheric pollution, are constituted by the combination of two or more of the aforementioned anti-glycosylation agents, vitamin C and/or its derivatives, and, optionally, vitamin E and/or its derivatives, and vitamin A and/or its derivatives and/or oils extracted from vegetable sources such as Helianthus annuus and Hedera helix and/or phytic acid for example extracted from the bran of rice.

In this respect, the invention relates to a dermatological and cosmetic composition as defined previously and which contains, in addition, either vitamin E and/or one or a plurality of its derivatives, or vitamin A and/or one or a plurality of its derivatives.

The invention also relates to the local or topical application of the composition of the invention as well as a method for treating symptoms of skin ageing, consisting in applying locally to the skin and for the prevention of the consequences of exposure to atmospheric pollution on the areas of the body of a mammal to be treated, an effective quantity of one of the aforedescribed compositions

The invention also relates to the use of these compositions for the preparation of a medicinal product for treating the symptoms of skin ageing and for the prevention of the consequences of exposure to atmospheric pollution.

Further advantages and characteristics of the invention will be understood more clearly from the following description and by reference to the figures therein:

Figure 1 gives the anti-glycosylation activity of methylsilanol mannuronate (Algisium C),

Figure 2 gives the anti-glycosylation activity of an extract of hydrolysed wheat protein (Integrissyme),

Figure 3 gives the anti-glycosylation activity of the combination of methylsilanol mannuronate (Algisium C) and an extract of hydrolysed wheat protein (Integrissyme),

Figure 4 gives the effect of various dermatological compositions on the synthesis of collagen by cultures of human fibroblast cells, and

Figure 5 gives the effect of various dermatological composition the glycosylation of the collagen produced by cultures of human fibroblast cells

Detailed description of the invention

Methylated silanol derivatives

Several compounds can be used as methylated silanol derivatives, including the following compounds, the list herebelow not being complete:

sodium mannuronate methylsilanol (Algisium, Exsymol)
methylsilanol mannuronate (Algisium C®, Exsymol)
methylsilanol mannuronate Nylon-12 (Algisium C powder®, Exsymol)
ascorbylmethylsilanol (Ascorbosilane concentrate C®, Exsymol)
ascorbylmethylsilanol pectinate (Ascorbosilane C®, Exsymol)
dimethyl oxobenzodioxsilane (DSBC®, Exsymol)
dimethyl oxobenzodioxasilane Nylon-12 (DSBC powder®, Exsymol)
sodium hyaluronate dimethylsilanol (DSH®, Exsymol)
dimethylsilanol hyaluronate (DSHC®, Exsymol)
methysilanol glycyrrhizinate (Glysinol®, Exsymol)
methylsilanolhydroxyproline (Hydroxyprolisilane®, Exsymol)
methylsilanolhydroxyproline aspartate (Hydroxyprolisilane C®, Exsymol)
sodium lactate methylsilanol (Lasilium®, Exsymol)
lactoylmethylsilanol elastinate (Lasilium C®, Exsymol)
dioleyl tocopheryl methylsilanol (Liposiliol C®, Exsymol)
methylsilanol acetylmethionate (Methiosilane®, Exsymol)
acetylmethionylmethylsifanol elastinate (Methiosilane C®, Exsymol)
methylsilanol PEG 7 glyceryl cocoate (Monosiliol®, Exsymol)
methylsilanol tri PEG 7 glyceryl cocoate (Monosiliol C®, Exsymol)
methylsilanol elastinate (Proteosilane C®, Exsymol)
pyrollidone carboxylate caustic methylsilanol (Silhydrate®, Exsymol)
pyrollidone carboxylate copper methylsilanol (Silhydrate C®, Exsymol)
methylsilanolcarboxymethyl theophylline (Theophyllisilane®, Exsymol)
methylsilancarboxymethyl theophylline alginate (Theophyllisilane C® Exsymol)
methylsilanol acetyltyrosine (Tyrosilane®, Exsymol), or
copper acetyl tyrosinate methylsilanol (Tyrosilane C® , Exsymol).

Among the derivatives of methylated silanol preferred in the context of this invention, are
sodium mannuronate methylsilanol,
methylsilanol mannuronate,
ascorbylmethylsilanol,
ascorbylmethylsilanol pectinate,
methylsilanol hydroxyproline,
methylsilanol hydroxyproline aspartate, or
methylsilanol acetyltyrosine .
A particularly preferred derivative of methylsilanol is methylsilanol mannuronate.
The above identified methylsilanol derivatives are commercially available from the sources indicated. For example, methylsilanol mannuronate is commercially available from Exsymol under the trade name Algisium C. This commercial product is an aqueous solution containing 1% methylsilanol mannuronate.
The amount of the commercial product containing the derivative of methylated silanol which may be included in the formulations of the present invention generally vary between 1 and 20 percent by weight, the preferred concentrations normally ranging between 2 and 7 percent by weight of the total weight of the composition. The compositions will therefore contain 0.01 to 0.2%, preferably 0.02 to 0.07%, of the methylated silanol derivative.

Derivatives of hydrolysed plant proteins

Several derivatives of hydrolysed plant proteins, more particularly hydrolysed plant proteins of cereal origin (for example barley, wheat, oats) can be used in combination with the derivative of methylated silanol to form the compositions of the invention. The choice of this derivative can be made easily by the person skilled in the art. These products include extracts of wheat proteins hydrolysed by an enzyme and containing two peptide groups of different molecular weight.

Suitable derivatives of hydrolysed plant proteins are commercially available. For example, a hydrolysed wheat protein is commercially available under the trade name Integrissyme. This commercial product is an aqueous preparation containing hydrolysed wheat protein (20%) polysorbate 20 (5%) and glycerine (5%).

The amount of the commercial product containing the hydrolysed plant protein generally varies between 0.25 and 5 percent by weight of the total weight of the composition, the preferred concentrations ranging between 0.5 and 3 percent by weight. The compositions will therefore contain 0.05 to 1%, preferably 0.1 to 0.6% of hydrolysed plant protein.

Vitamin C

Vitamin C and its derivatives has an effect on the synthesis and the secretion of collagen outside the cells. In this process, vitamin C has two major functions:-

(1) vitamin C is the cofactor of the two enzymes, lysyl and prolyl hydroxylase, which are responsible for the hydroxylation of tropocollagen intended to initiate its secretion outside the cell (Freiberger et al 1980, Murad et al 1981 and 1983. Tajima and Pinnell 1982).

(2) vitamin C controls the replication of three genes ($proa_1$, $proa_2$ and $proa_3$) positioned on different chromosomes to initiate the biosynthesis of collagen (Pinnell et al 1987).

The combination of vitamin C and of its derivatives (for example, magnesium ascorbyl phosphate) with the composition of the invention can further improve the anti-glycosylation agents to improve the method of anti-ageing treatment, since, with these compounds, it is possible to stimulate the synthesis of the collagens of the connective tissue while protecting them against non-enzymatic glycosylation.

It has been established by *in vitro* biochemical tests and cell culture tests that the use of a combination of the aforementioned compounds particularly methylsilanol mannuronate, hydrolysed plant proteins and, optionally, magnesium ascorbyl phosphate, in an aqueous solution or in creams in the amount effective in treating the symptoms of skin ageing.

Hydrolysed vitamin C or ascorbic acid can be extracted from plants or synthesized chemically. It possesses relatively high reducing power and, in an aqueous solution, it is very sensitive to oxidation in the presence of molecular oxygen, alkalis, metal and in certain pH conditions. This is why it is preferable when this substance is used in its molecular form, that it is in acidic pH conditions preferably between 2.5 and 4.

Furthermore, more stable derivatives of the substance also exist, such as ascorbyl salts or ascorbyl esters and the encapsulated forms of vitamin C: magnesium ascorbate (Vitacedone® UCIB) or magnesium ascorbyl phosphate (Nikkol VC-PMG®, Jan Dekker) or ascorbyl and disodium sulphate (Nikkol VC-SS®, Jan Dekker) or ascorbyl palmitate or ascorbic acid polypeptide (Vitazyme C®, Brooks) or ascorbylmethylsilanol pectinate (Ascorbilane®, Exsymol) or microspheres whereof the wall is made of carraghenine encapsulating vitamin C (Lipotec) or microspheres whereof the wall is made of atelocollagen encapsulating magnesium ascorbyl phosphate (Thallaspheres Coletica). If, for example, magnesium ascorbyl phosphate is used, the pH of the aqueous phase is preferably between 7 and 8, for the stability of the substance in aqueous medium.

The use of at least one of the aforementioned forms of vitamin C or of its derivatives or of its encapsulated forms, in an aqueous solution or in a cream in the amount of 0.25 to 30 percent is effective. If two different forms or more of vitamin C are used in combination, it is preferable for the total concentration of the compounds not to exceed 20 percent by weight of the composition.

Vegetable extracts

The vegetable sources from which suitable oils can be obtained include Helianthus annuus (sunflower) and Hedera helix. Phytic acid can be extracted from the bran of rice. The compositions of the present invention may contain 0.005 to 0.05%, preferably 0.01 to 0.04%, of each of oil extracted from Helianthus annuus, oil extracted from Hedera helix and phytic acid extracted from the bran of rice. An aqueous composition containing an extract of Helianthus annuus (3%), an extract of Hedera helix (2%) phytic acid from the bran of rice (2%), glycerin (40%), PEG-8 (15%), caprylyl glycol (4.5%), PPG-1/PEG-9/lauryl glycol ether (3%), butylene glycol (3%) and sodium polyacrylate (0.5%) is available commercially from Sederma under the trade name Osmopur. This commercial composition may be used in the compositions of the present invention at a level of 0.5 to 2%, preferably around 1% of the total composition.

Oligopeptides and derivatives

The oligopeptide or derivative is preferably palmitoyl oligopeptide. This material is present in commercially available materials such as:

Biopeptide CL (Sederma) which contains glyceryl polymethacrylate, propylene. glycol and a palmitoyl oligopeptide in which the oligopeptide is composed of glycine, histidine and lysine moieties.

Biopeptide FN (Sederma) which contains glyceryl polymethacrylate, butylene glycol and a palmitoyl oligopeptide in which the oligopeptide is composed of arginine, glycine, aspartic acid and serine moieties.

Amadorine (Solavia) which is a polypeptide composed of arginine and lysine moieties.

Citogard (Pentapharm) which is a saccharomyces polypeptide.

The amount of the commercial product (eg Biopeptide CL) which may be included in the formulations of the present invention generally varies between 1 and 20%, preferably 2 to 7% by weight of the total weight of the composition.

Vitamin E

Vitamin E and its derivatives can be used in several forms, the choice of which can be made without difficulty by the person skilled in the art. For example, DL-tocopherol or tocopherol acetate are preferred.
The concentration of vitamin E in the compositions of this invention generally varies between 0.05 and 2% by weight of the total weight of the composition.

Vitamin A

Vitamin A and its derivatives can also be used in several forms the choice of which can be made without difficulty by the person skilled in the art. For example, retinol, retinyl acetate or retinyl palmitate are preferred.
The concentration of vitamin A in the compositions of this invention generally varies between 0.02 and 1 percent by weight of the total weight of the composition.

Formulation of the composition

The combination of the aforementioned compounds can be incorporated, preferably, in a water-in-oil emulsion (including a water-in-silicone oil emulsion), in an oil-in-water emulsion or in a multiple water-in-oil-in-water emulsion or in a pseudo-emulsion (dispersion of two immiscible phases, an oily phase in an aqueous phase, using thickening agents).
Preferably, the compositions of this invention are in the form of either a water-in-oil emulsion, an oil-in-water emulsion, or a multiple water-in-oil-in-water emulsion or a pseudo-emulsion. Such emulsions or pseudoemulsions may comprise the following materials:-

(a) Oily phase

The oily phase of the emulsions of this invention can contain, for example:

hydrocarbon oils such as paraffin or mineral oils such as isohexadecane,

natural oils such as sunflower oil, primrose oil, jojoba oil, hydrogenated caster oil, avocado oil or hydrogenated palm oil,

natural triglycerides such as caprylic/capric triglyceride,

caprylic/capric/linoleic triglyceride or caprylic/capric/succinic triglyceride,

silicone oils such as cyclomethicone, dimethicone or dimethiconol,

fatty acid esters such as myristyl myristate or isopropyl myristate,

fatty alcohols such as hexadecyl alcohol or stearyl alcohol,

waxes such as beeswax, paraffin, carnauba wax or ozokerite,

lanolin and its derivatives (oil, alcohol, waxes) , or

mixtures thereof.

In the particularly preferred water-in-oil emulsion, or the oil-in-water emulsion or other media comprising the composition of this invention, the oily phase represents from about 5 to about 30 percent, and preferably from about 10 to about 20 percent by weight of these compositions.

(b) Emulsifiers

The emulsifiers which can be used in the compositions of this invention can be selected from among the emulsifiers known in the art and usable in water-in-oil or oil-in-water emulsions.

The water-in-oil and oil-in-water compositions can be prepared by using an emulsifier selected from among the emulsifiers acceptable for cosmetics including:

sesquioleates such as sorbitan sesquioleate.

emulsifiers based on silicon oil such as silicone polyols,

sorbitan esters and ethoxylated sorbitan esters such as sorbitan stearate or polysorbate,

glyceryl esters such as glyceryl stearate or glyceryl isostearate,

sucroesters such as saccharose cocoate and saccharose distearate,

ethoxylated fatty alcohols such as ethoxylated hexadecyl alcohol or ethoxylated stearyl alcohol,

ethoxylated soya sterols, or

mixtures of the aforementioned emulsifiers.

The quantity of emulsifier that may be present in the oil-in-water, water-in-oil composition is, preferably, in the range from about 0.5 to about 15 percent by weight of said composition. The quantity of emulsifier that may be present in the multiple water-in-oil-in-water emulsion of this invention is preferably, in the range from about 7 to about 20 percent by weight of said composition.

(c) Other components of the formulation

The compositions of this invention can furthermore comprise one or more other compounds which are known to the specialists in the art, for example:

electrolytes for stabilizing emulsions such as sodium chloride or magnesium sulphate or sodium citrate, preferably in a quantity ranging from about 0.2 to about 4 percent by weight of said composition,

humectants such as glycerine, propylene glycol, polyethylene glycol (PEG) or sorbitol, preferably in a quantity ranging from about 1 to about 10 percent by weight of said composition,

thickeners such as xanthan gum, derivatives of cellulose, carbomers, copolymers of acrylic acid, gum sclerosium, preferably in a quantity ranging from about 0.05 to about 1 percent by weight of said composition,

chelatants such as tetrasodium EDTA, preferably in a quantity ranging from about 0.01 to about 0.5 percent by weight of said composition,

softening agents such as fatty acid ether or fatty acid ester, preferably in a quantity ranging from about 0.5 to about 10 percent by weight of said composition,

hydrating agents such as D-panthenol, hyaluronic acid, sodium pyrrolidone carboxylate, preferably in a quantity ranging from about 0.01 to about 5 percent by weight of said composition,

film-forming agents to facilitate the spreading on the surface of the skin, such as polymethacrylates, preferably in a quantity ranging from about 0.05 to about 3 percent by weight of said composition,

organic sun blockers such as octyl methoxycinnamate, butyl methoxydibenzoylmethane, isoamyl methoxycinnamate, octyl dimethyl PABA, octyl salicylate, benzophenone 3, octyl triazone, ethyl 4-polyethoxy-5-aminobenzoate, isopropyl 4-dibenzoyl-methane, 2-phenyl-benzimidazol-5-sulphonic acid, 2-hydroxy-4-methoxy-benzophenone-5-sulphonic acid, preferably in a quantity ranging from about 0.5 to about 5 percent by weight of said composition,

insoluble pigments such as titanium dioxide, rutile titanium dioxide, anatase titanium dioxide,

inorganic sunscreens for example pyrogenic microfine titanium dioxide such as P 25®, Degussa, microfine titanium dioxide such as Sun Veil® Ikeda, microfine titanium dioxide surface-treated by silicones, or by amino acids, or by lecithin, or by metallic stearates, microfine iron oxide, iron oxide surface-treated by

silicones, or by amino acids, or by lecithin, or by metallic stearates, zinc oxide, microfine zinc oxide like UFZO® (Cosmo Trends Corporation), mica coated with titanium dioxide, preferably in a quantity ranging from about 0.5 to about 5 percent by weight of said composition.

preservatives such as methyl p-hydroxybenzoate, propyl p-hydroxybenzoate, phenoxyethanol, 2-bromo-2-nitropropane-1,3-diol or mixtures thereof, preferably in a quantity ranging from about 0.05 to about 3 percent by weight of said composition,

perfumes, preferably in a quantity ranging from about 0.05 to about 0.6 percent of said composition,

colorants, preferably in a quantity ranging from a trace to about $3 \times 10^{-3}$ percent by weight of said composition, or

mixtures thereof.

The invention is illustrated by the following Examples which are given by way of example only. In the Examples that follow the amounts of each component are expressed as percentages by weight of the total composition.

Example 1: night cream (water-in-silicone oil emulsion)

Table 1

| Composition of the oily phase | |
| --- | --- |
| Ingredient | % |
| Cyclomethicone | 5 |
| Cyclomethicone/dimethicone copolyol | 10 |
| Dimethicone and dimethiconol | 5 |
| Myristyl ether of PPG-3 | 1.5 |
| Tocopherol acetate | 0.5 |
| Retinyl palmitate (Note 1) | 0.15 |
| Dimethicone of behenic ester | 1 |

Table 2

| Composition of the aqueous phase | |
| --- | --- |
| Ingredient | % |
| Tetrasodium EDTA | 0.05 |
| Sodium chloride | 0.8 |
| Glycerine | 5 |
| PEG-8 | 1.5 |
| Methyl p-hydroxybenzoate | 0.25 |
| Propyl p-hydroxybenzoate | 0.15 |
| Phenoxyethanol | 1 |
| Magnesium ascorbyl phosphate (Note 2) (Nikkol VC-PMG) | 1 |
| Aqueous preparation containing hydrolysed wheat protein (20%) polysorbate 20 (5%) and glycerine (5%) (Note 2) (Integrissyme) | 0.5 |
| 1 % Aqueous solution of methylsilanol mannuronate (Note 2) (Algisium C) | 3 |
| Red FD&C No. 40 | $1.5 \times 10^{-3}$ |
| Perfume (Note 2) | 0.15 |
| Water | qs |
| Notes to Tables 1 and 2<br>(1) It is preferable not to heat the retinyl palmitate with the other compound of the oily phase to prevent its oxidation. It is preferable to incorporate it in the phase at the beginning of emulsification.<br>(2) Heat-sensitive compound. | |

The oily and aqueous phases, with the exception of the aforementioned heat-sensitive compounds, were heated to 50 ±1°C separately. They were then mixed with strong stirring by a microvortex type stirrer (Rayneri®, France). Stirring was maintained at constant speed until the temperature of the emulsion dropped to 30°C. At this stage, the homogenization speed was reduced and the heat-sensitive ingredients were incorporated. Emulsification was continued until the cream was completely homogeneous.

Example 2: Day cream (multiple water-in-oil-water emulsion)

Table 3

| Composition of the primary emulsion | |
| --- | --- |
| Ingredient | % |
| Oily phase | |
| Isohexadecane | 2 |
| Cetyl octanoate | 5 |
| Cyclomethicone | 3.5 |
| Cetyldimethicone copolyol | 4.2 |
| Octyl methoxycinnamate | 2 |
| Butyl methoxydibenzoylmethane | 0.5 |
| Tocopherol acetate | 0.5 |
| Retinyl palmitate | 0.15 |
| | |
| Aqueous phase | |
| Sodium citrate | 0.4 |
| Magnesium sulphate | 0.5 |
| Tetrasodium EDTA | 0.1 |
| Xanthan gum | 0.1 |

Table 3   (continued)

| Composition of the primary emulsion | |
| --- | --- |
| Ingredient | % |
| Aqueous phase | |
| Magnesium ascorbyl phosphate (Nikkol VC-PGM) | 1 |
| Panthenol | 1 |
| Propylene glycol | 2.5 |
| PEG-8 | 2 |
| Methyl p-hydroxybenzoate | 0.2 |
| Propyl p-hydroxybenzoate | 0.1 |
| 2-bromo-2-nitropropane-1,3-diol | 0.05 |
| Perfume | 0.15 |
| Water | 44 |

Table 4

| Composition of the external aqueous phase | |
| --- | --- |
| Ingredient | % |
| Sodium hyaluronate | 0.03 |
| PEG-8 | 2 |
| Methyl p-hydroxybenzoate | 0.2 |
| Propyl p-hydroxybenzoate | 0.1 |
| 2-Bromo-2-nitropropane-1,3-diol | 0.05 |
| Polyglycerylmethacrylate/propylene glycol/ PVM/MA copolymer | 5 |
| Red FD&C No. 40 | $1.5 \times 10^{-3}$ |
| Ceteth-20 | 2.1 |
| Soya sterol PEG-25 | 0.9 |
| 1 % Aqueous solution of methylsilanol mannuronate (Algisium C) | 3 |
| Aqueous preparation containing hydrolysed wheat protein (20%) polysorbate 20 (5%) and glycerine (5%) (Integrissyme) | 0.5 |
| Polymethylmethacrylate | 0.8 |
| Water | qs |

The multiple emulsion was prepared by using the two-step emulsification method as described previously in Tokgoz 1996. In this method the primary water-in-oil emulsion was prepared and said emulsion was dispersed in the external aqueous phase containing hydrophilic emulsifiers, a thickening agent, active agents and other ingredients. In the first step of emulsification to prepare the primary water-in-oil emulsion, the phases (oily and aqueous phases) were heated and mixed as in Example 1. However, in the second step, the primary emulsion was incorporated drop by drop (incorporation time = 20 min) in the external phase at ambient temperature and stirring was carried out at very low speed (homogenization time = 10 min).

Example 3: Day cream (oil-in-water emulsion containing a liquid crystal phase)

Table 5

| Composition of the oily phase | |
| --- | --- |
| Ingredient | % |
| Cyclomethicone | 5 |
| Dioctyl succinate | 3 |
| Polyacrylamide (45%)/C13,C14 isoparaffin (25%)/laureth-7(8%) (Sepigel 305) | 3 |

Table 5   (continued)

| Composition of the oily phase | |
|---|---|
| Ingredient | % |
| Hydrogenated lecithin | 2 |
| Tocopherol acetate | 0.5 |
| Retinyl palmitate | 0.15 |
| Palmitic acid | 1 |
| C12, C16 alcohols | 1 |

Table 6

| Composition of the aqueous phase | |
|---|---|
| Ingredient | % |
| Nylon-12 | 2 |
| Gum Sclerotium | 0.3 |
| Tetrasodium EDTA | 0.05 |
| PEG-8 | 1.5 |
| Methyl p-hydroxybenzoate | 0.25 |
| Propyl p-hydroxybenzoate | 0.15 |
| O-Cymen-5-ol | 0.1 |
| Magnesium ascorbyl phosphate (Nikkol VC-PGM) | 1 |
| An aqueous preparation containing hydrolysed wheat protein (20%) polysorbate 20 (5%) and glycerine (5%) (Integrissyme) | 0.5 |
| 1% Aqueous solution of methylsilanol mannuronate (Algisium C) | 3 |
| Red FD&C No. 40 | $1.5 \times 10^{-3}$ |
| Perfume | 0.40 |
| Water | qs |

The same method of preparation was used as for the preparation of Example 1.

Example 4: Body lotion (oil-in-water emulsion)

Table 7

| Composition of the oily phase | |
|---|---|
| Ingredient | % |
| Isohexadecane | 8 |
| Mineral oil | 5 |
| Sorbitan stearate | 2 |
| Ceteth 20 | 2 |
| Tocopherol acetate | 0.5 |
| Retinyl palmitate | 0.15 |
| Cyclomethicone | 1 |
| Hexadecyl alcohol | 0.5 |

Table 8

| Composition of the oily phase | |
|---|---|
| Ingredient | % |
| Glycerine | 5 |
| Propylene glycol | 5 |
| Methyl p-hydroxybenzoate | 0.25 |
| Propyl p-hydroxybenzoate | 0.15 |
| Sodium carbomer | 0.35 |
| Magnesium ascorbyl phosphate (Nikkol VC-PGM) | 1 |
| An aqueous preparation containing hydrolysed wheat protein (20%) polysorbate 20 (5%) and glycerine (5%) (Integrissyme) | 0.5 |
| 1% Aqueous solution of methylsilanol mannuronate (Algisium C) | 3 |
| Red FD&C No. 40 | $1.5 \times 10^{-3}$ |
| Perfume | 0.15 |
| Water | qs |

The same method of preparation was used as for the preparation of Example 1.

Example 5: day cream (oil-in-water emulsion without emulsifier)

Table 9

| Composition of the oily phase | |
|---|---|
| Ingredient | % |
| Isohexadecane | 15 |
| Myristyl myristate | 3 |
| Beeswax | 1.5 |
| Squalane | 0.075 |
| Titanium dioxide | 1 |
| Tocopherol acetate | 0.5 |
| Retinyl palmitate | 0.15 |
| Soya sterol | 0.5 |
| Macadamia oil | 0.2 |
| Cyclomethicone | 2 |
| Hexadecyl alcohol | 1.5 |

Table 10

| Composition of the aqueous phase | |
|---|---|
| Ingredient | % |
| Polyglycerylmethacrylate | 4.8 |
| Propylene glycol | 0.12 |
| PEG-8 | 3 |
| Methyl p-hydroxybenzoate | 0.25 |
| Propyl p-hydroxybenzoate | 0.15 |
| O-Cymen-5-ol | 0.1 |
| 2-Bromo-2-nitropropane-1,3-diol | 0.05 |
| Tetrasodium EDTA | 0.05 |
| Sodium carbomer | 0.35 |

Table 10   (continued)

| Composition of the aqueous phase | |
|---|---|
| Ingredient | % |
| Magnesium ascorbyl phosphate (Nikkol VC-PGM) | 1 |
| An aqueous preparation of hydrolysed wheat protein (20%) polysorbate 20 (5%) and glycerine (5%) (Integrissyme) | 0.5 |
| 1% Aqueous solution of methylsilanol mannuronate (Algisium C) | 3 |
| Red FD&C No. 40 | $1.5 \times 10^{-3}$ |
| Perfume | 0.15 |
| Water | qs |

The same method of preparation was used as for the preparation of Example 1.

Example 6

Table 11

| Composition of the oily phase | |
|---|---|
| Ingredient | % |
| Caprylic/Capric triglyceride | 3 |
| Cetearyl octanoate | 4 |
| Stearic acid | 1.5 |
| Cetyl alcohol | 1 |
| Glyceryl stearate | 2 |
| Cyclomethicone | 2 |
| Tocophenyl acetate | 0.5 |
| Retinyl palmitate | 0.15 |
| PEG-10 Soya sterol | 1 |
| Beeswax (Cera alba) | 1.2 |
| Cetyl palmitate | 3 |

Table 12

| Composition of the aqueous phase | |
|---|---|
| Ingredient | % |
| Tetrasodium EDTA | 0.05 |
| Methyl gluceth-20 | 3 |
| Glycereth-26 | 2 |
| Acrylates/C10-30 alkylacrylate crosspolymer (Pemuten TRI) | 0.2 |
| Biosaccharide gum-1 (Fucogel 1000 pp) | 5 |
| PEG-8 | 1.5 |
| Methyl p-hydroxybenzoate | 0.3 |
| Propyl p-hydroxybenzoate | 0.2 |
| O-Cymen-5-ol | 0.1 |
| Phenoxyethanol | 0.90 |
| Sodium hyaluronate | 0.1 |
| Sodium hydroxide | 0.22 |
| Magnesium ascorbyl phosphate (Nikkol VC-PMG) | 1 |
| Glycerin | 3 |

Table 12   (continued)

| Composition of the aqueous phase | |
| --- | --- |
| Ingredient | % |
| Aqueous preparation containing hydrolysed wheat protein (20%), polysorbate 20 (5%) and glycerin (5%) (Integrissyme) | 0.5 |
| 1% Aqueous solution of methylsilanol mannuronate (Algisium C) | 3 |
| An aqueous preparation containing glycerin (40%), PEG-18 (15%), Caprylyl glycol (4.5%), extract of Helianthus annuus (3%), PPG-1-PEG-9-lauryl glycol ether (3%), butylene glycol (3%), extract of Hedera helix (2%), phytic acid extracted from the bran of rice (2%) and polyacrylate (0.5%) (Osmopur) | 1 |
| Perfume | qs |
| Water | qs |

The same method of preparation was used as for the preparation of Example 1.

*In vitro* glycosylation inhibition tests

The derivatives of methylated silanol (Algisium C®), hydrolysed wheat proteins (Integrissyme®) and the compositions of the invention comprising these derivatives were initially subjected to a series of biochemical analyses *in vitro* to determine their effectiveness. The test protocol used is known in the literature (Rosenberg et al 1979, Schinder and Kohn 1980) and is a simple test designed to induce non-enzymatic giycosylation of the proteins in laboratory conditions.

The *in vitro* analysis employed consists in incubating beef serum albumin (BSA) and D-glucose and a sample of the active compound in a phosphate buffer (pH 7.4) at 37 °C for three weeks. This incubation gives a BSA with cross linkages. Once glycosylated, the BSA is subjected to an acidic hydrolysis reaction to liberate the reactive carbonyl groups of 5-hydroxymethylfuraldehyde (HMF). After precipitation and removal of the BSA from the medium, the addition of thiobarbituric acid (TBA) causes a coloration reaction to determine the quantity of amine/hexose bonds which is proportional to the quantity of glycosylated proteins. The effective anti-glycosylation compounds reduce the quantity of free HMF in the medium or, in other terms, the amount of glycosylation.

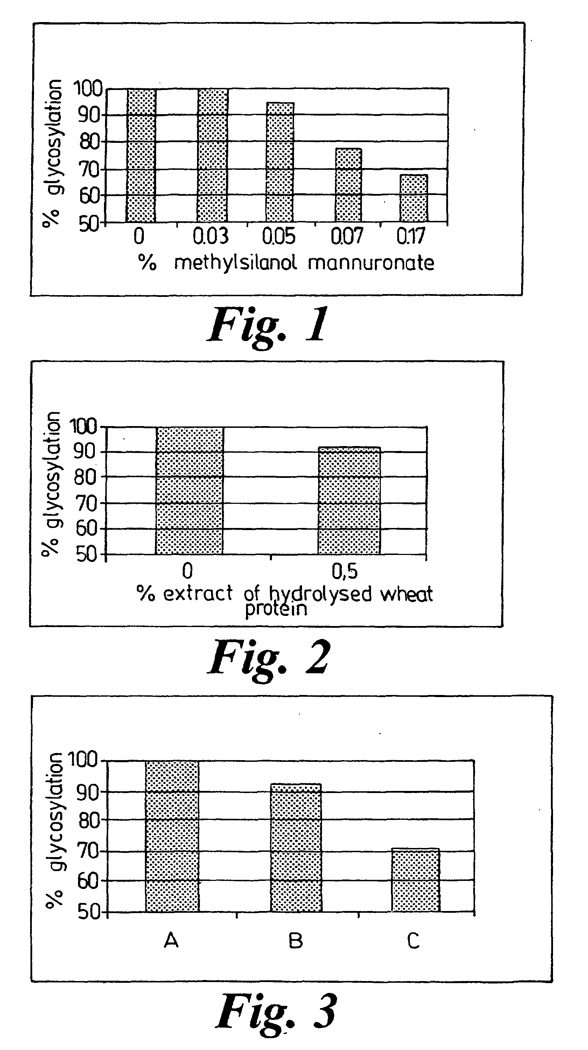

The results of these tests are given in Figures 1 to 3. In these figures the percentage glycosylation is shown as a function of the concentration of the active compound used. For Figure 1, the active material is methylsilanol mannurate (Algisium C) used alone. For Figure 2, the active material is hydrolysed wheat protein extract (Integrissyme) used alone. Figure 3 gives the results obtained using compositions comprising methylsilanol mannurate alone (Algisium C concentration 3 percent), or hydrolysed wheat protein extract alone (Integrissyme concentration 0.5 percent), or a mixture of methylsilanol mannurate (Algisium C concentration 3 percent) and hydrolysed wheat protein extract (Integrissyme ,concentration 0.5 percent).

As shown in Figures 1,2 and 3, the combination of the two anti-glycosylation agents as described in the present application appears to prevent glycosylation more effectively than if only one compound thereof is used in large quantities

In fact, the anti-glycosylation activity of hydrolysed wheat protein extract (Integrissyme®) appears to be higher than that of methylsilanol mannuronate (Algisium C®) when it is tested alone. With Algisium C®, the only significant decrease in glycosylation was obtained with concentrations higher than 7 percent. Algisium C® proved to be ineffective when used in a concentration of 3 percent. However, the combination of Algisium C® (3 percent) with Integrissyme® (0.5 percent) significantly reduced the free HMF in the medium.

Effect of compositions of the invention on the synthesis of collagen *in vivo*

The effect of a composition of the present invention (comprising two anti-glycosylation agents with magnesium ascorbyl phosphate) on the stimulation of the synthesis of collagen and the limitation of the formation of irreversible cross linkages between the collagen fibrils, were evaluated *in vitro* on human fibroblast cells.

The cells used were normal human fibroblasts (NHDF 784) used in the fourth passage (R4) and cultured at 37 °C, in 5 percent CO2 atmosphere, in the following culture medium: MEM/M199, 3/1 (Gibco 31570021/2115130), sodium bicarbonate (Gibco 25080060) 1.87 mg/ml, L-glutamine (Gibco 25030024) 2 mmol/l, penicillin (Polylabo 60703) 50 UI/ml, and foetal calf serum (v/v Gibco 10106151) 10 percent.

Six preparations in sterile culture medium were made as follows:-

preparation 1 :vitamin C (1 mg/ml) (Vit-C),

preparation 2:magnesium ascorbyl phosphate (Nikkol VC-PMG -1 percent) (Vit-C PMG),

preparation 3:D-glucose (1 percent),

preparation 4:magnesium ascorbyl phosphate (Nikkol VC-PMG -1 percent) + methylsilanol mannuronate (Algisium C -3 percent) (Mixture 1),

preparation 5: magnesium ascorbyl phosphate (Nikkol VC-PMG -1 percent) + hydrolysed wheat protein (Integrissyme -0.5 percent) (Mixture 2), and

preparation 6: magnesium ascorbyl phosphate (Nikkol VC-PMG -1 percent) + methylsilanol mannuronate (Algisium C - 3 percent) + hydrolysed wheat protein (Integrissyme - 0.5 percent) (Mixture 3),

The fibroblasts were distributed in four plates of twelve wells (two plates for the synthesis of collagen, two plates for glycation) at the rate of 9x104 cells/well and cultured for 24 h before distribution of the products (> 80 percent confluence).

The mixtures used in the study were in 1/40 dilutions in these stock solutions prepared in the sterile culture medium. They were non-cytotoxic doses, according to the preliminary tests. Vitamin C was tested in the final concentration of 20 $\mu$g/ml (113 $\mu$mol/l). This concentration is optimal to stimulate the synthesis of procollagen *in vitro* (Freiberger et al 1980). Glucose was tested in a final concentration of 0.1 percent, which is equivalent to doubling the quantity of 'cold' glucose of the medium. Each experimental condition was carried out in triplicate (three culture wells). The control wells received 1.2 ml of culture medium alone.

Incubation lasted 72h, with renewal of the test media at 24h intervals. Metabolic labelling took place during the last 24h with 40 $\mu$Ci per well (33.3 $\mu$Ci/ml) of L-[2,3-$^3$H]-proline, 44 Ci/mmol (16.3 TBq/mmol, Amersham, TRK628) or D-[5-$^3$H]-glucose, 14.3 Ci/mmol (503 GBq/mmol, Amersham TRK290).

At the end of incubation, the plates containing the culture medium were subjected to a freeze/thaw cycle. The medium of each well (1.2 ml) was sampled, and each well was washed twice with 1 ml of iced water. The culture media and the wash solutions of each well were added together. They contained free radioactivity and radioactivity incorporated in the soluble cellular and extracellular proteins. The bottom of each well containing the insoluble material was again washed and the plates were stored in ice. The proteins of the soluble fraction were then precipitated by 0.3 volume of 10 percent w/v trichloroacetic acid (TCA), and then washed twice with 3 percent TCA (removal of free radioactivity). The proteins were purified by centrifugation on 42 individual columns (Micro-spin G-25, Pharmacia). This operation serves to remove any remaining traces of free radioactivity and to remove the TCA present in the samples (collagenase inhibitor).

The samples of soluble and insoluble proteins were then subjected to digestion by five ultrapure collagenase units (Sigma, C0775) in a Tris-HCl 50 mmol/l, CaCl$_2$ 5 mmol/l buffer (final concentrations), for 3 h at 37 °C. The collagenase used contained 1390 collagenase units/mg for 0.2 unit/mg of neutral protease activity (caseinase). Aliquots were taken to count the incorporated radioactivity in the proteins and a final precipitation was carried out with TCA to separate the material digested by collagenase (collagenic origin) from the collagenase-insensitive material. The incorporated radioactivity was counted by liquid scintillation after cumulation of the soluble/insoluble protein fractions, in an LKB 1211 Rackbeta counter.

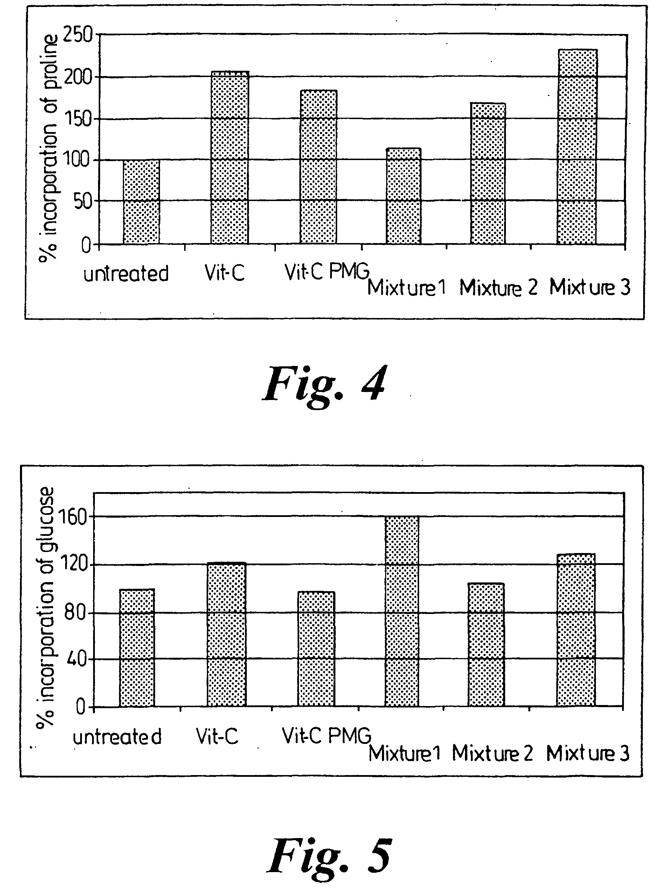

The results of these tests show that the combination of the two anti-glycosylation agents (Algisium C®) and Integrissyme®) with magnesium ascorbyl phosphate very significantly augments the synthesis of new collagen molecules, that is the incorporation of radiolabelled proline in the newly synthesized collagen molecules (Figure 4). Furthermore, this event was not accompanied by an increase in glycosylated collagen in the medium, that is the incorporation of radiolabelled glucose in the collagen matrix (Figure 5).

It appears in Figure 4 that vitamin C (alone), magnesium ascorbyl phosphate (alone), Mixture 2 and Mixture 3 stimulated the synthesis of collagen, that is the quantity of radiolabelled 'proline' in the neosynthesized collagen was significantly increased. The best results were obtained with the combination of the two anti-glycosylation agents and magnesium ascorbyl phosphate (mixture 3).

In contrast, mixture 1 had practically no effect on the stimulation of collagen synthesis, and this led to an increase in the quantity of irreversible cross linkages formed between the collagens (Figure 5). These results could be explained by a synergetic effect between two anti-glycosylation agents preferably combined with magnesium ascorbyl phosphate.

In conclusion, the neosynthesis of collagen induced by mixture 3 was not accompanied by an increase in the glycation rate. In other words, the glycation rate remains the same for different quantities of collagen. Nevertheless, mixture 3 does not inhibit glycation strictly speaking, hence we can infer an important effect on the neosynthesis of collagen and a control (with limitation) of glycation.

Anti-pollution activity of compositions of the present invention

The anti-pollution activity of the compositions of the present invention was demonstrated in the following test.

Twelve female volunteers aged between 21 and 57 took part in the test. Three zones were selected on the forearm of each volunteer. To one zone the product of Example 6 was applied at a rate of 2μl/cm$^2$. To a second zone a composition similar to that of Example 6 except that it did not contain the methylsilanol mannuronate (Algisium C), the hydrolysed wheat protein (Integrissyme), the magnesium ascorbyl phosphate (Nikkol VC-PMG) or the aqueous preparation containing extracts of Helianthus annuus and Hedera helix and phytic acid (Osmopur) was applied at a rate of 2μl/cm$^2$. The third zone was untreated. After twenty minutes a suspension of carbon was applied to each zone at a rate of 2μl/cm$^2$. Forty minutes later the zones were rinsed with water, dried with a paper handkerchief and allowed to dry in the air. Ten minutes later each zone was stripped by application of adhesive tape to remove any carbon remaining on the skin within each zone. The adhesive tapes were then examined with a video microscope to determine the amount of carbon remaining in each zone. The results were expressed as a percentage protection P where P is calculated by the following formula

$$P = [(ZNT - ZT)/ZNT] \times 100$$

in which ZNT is the amount of carbon remaining in the non-treated zone and ZT is the amount of carbon remaining in the treated zone. The mean value of P calculated for the first zones of all the volunteers was 74% and the mean value of P calculated for the second zones was 57%. These results were statistically significant (p <0.001 by the student t-test). The higher the value of P the more effective the composition is in preventing the detrimental consequences of exposure to atmospheric pollution.

References

Lehninger A.L. Principles of Biochemistry Worth Publications Inc 3rd Edition, 1984

Cerami A., Vlassara H. and Brownlee M., Pour la Science, July 72-79 (1987)

Brownlee M., Vlassara H., Kooney A., Ulrich P. and Cerami A., Science, June, 1629-1631 (1986)

Shin D.H.. Hayase F. and Kato H., Agric.Biol.Chem., 52 1451-1458 (1988)

Igaki N., Yamada H., Oimomi M. and Kasuga M., Clinica Chimica Acta, 199 113-116 (1991)

Freiberger H., Grove D., Sivarajah A. and Pinnell S., J. Invest. Dermatol., 75 425-430 (1980)

Murad S., Sivarajah A- and Pinnell S., Biochem.Biophys.Res.Commun., 101 868-875 (1981)

Murad S., Tajima S., Johnson G.R., Sivarajah A. and Pinnell S., J. Invest. Dermatol., 81 158-162 (1983)

Pinnell S., Murad S. and Darr D., Arch.Dermatol., 123 1684-1687 (1987)

Tokgoz N.S., Doctoral Thesis in Pharmaceutical Sciences of the Universite de Paris-Sud, Paris XI, France, 1996

Rosenberg H., Modrak J.B., Hassing J.M., Al-Turk W.A. and Stohs S.J., Biochem Biophys Res Commun., 91 498-501 (1979)

Schinder S.L. and Kohn R.R., J.Clin.Invest., 66 1179-1181 (1980)

**Claims**

1. Dermatological and/or cosmetic composition for the treatment of symptoms of skin ageing comprising a combination of 0.01 to 0.2% by weight of the total composition of at least one derivative of methylated silanol and 0.05 to 1% by weight of the total composition of at least one derivative of hydrolysed plant protein which is hydrolysed barley protein, hydrolysed wheat protein or hydrolysed oat protein.

**2.** The composition of claim 1, wherein the derivative of methylated sitanol is methylsilanol mannuronate.

**3.** The composition of claim 1, wherein the derivative of hydrolysed plant protein is an extract of hydrolysed wheat protein.

**4.** The composition of any of claims 1 to 3, which further contains vitamin C and/or one or a plurality of its derivatives.

**5.** The composition of claim 4, wherein the derivative of vitamin C is magnesium ascorbyl phosphate.

**6.** The composition of any of claims 1 to 5 which further contains one or more oils extracted from a vegetable source and/or phytic acid.

**7.** The composition of claim 6 wherein the one or more oils are extracted from Helianthus annuus and/or Hedera helix and the phytic acid is extracted from the bran of rice.

**8.** The composition of any of claims 1 to 7 which further contains an oligopeptide or a derivative thereof.

**9.** The composition of claim 8 in which the derivative of the oligopeptide is a palmitoyl oligopeptide in which the. oligopeptide is composed of glycine, histidine and lysine moieties or arginine, glycine, aspartic acid and serine moieties; the oligopeptide is a polypeptide composed of arginine and lysine moieties or the oligopeptide is a saccharomyces polypeptide:

**10.** The composition of any of claims 1 to 9, which further contains vitamin E and/or one or a plurality of its derivatives.

**11.** The composition of claim 10, wherein the vitamin E or its derivative is DL-tocopherol or tocopheryl acetate.

**12.** The composition of any of claims 1 to 11, which further contains vitamin A and/or one or a plurality of its derivatives.

**13.** The composition of claim 12, wherein the vitamin A or its derivative is retinol, retinyl acetate or retinyl palmitate.

**14.** The composition of claim 4 or claim 5, wherein the vitamin C and/or its derivatives are present in a quantity from 0.25 to 30 percent by weight of the composition.

**15.** A composition as claimed in claim 6 or claim 7 wherein each of the one or more vegetable oils and/or the phytic acid is present in a quantity of from about 0.005 to 0.05% of the composition.

**16.** A composition as claimed in claim 7 wherein the oil extracted from Helianthus annuus is present in a quantity of from about 0.01 to 0.04% of the composition, the oil extracted from Hedera helix is present in a quantity from about 0.01 to 0.04% of the composition and phytic acid (if present) is present in a quantity of from about 0.01 to 0.04% of the composition.

**17.** The composition of claim 10 or claim 11, wherein the vitamin E and/or its derivatives are present in a quantity from about 0.05 to 2 percent by weight of the composition.

**18.** The composition of claim 12 or claim 13, wherein the vitamin A and/or its derivatives are present in a quantity from about 0.02 to 1 percent by weight of the composition.

**19.** The composition of any one of claims 1 to 18, **characterized in that** said composition is in the form of water/oil emulsion, a water/silicone oil emulsion, an oil/water emulsion, a multiple water/oil/water emulsion or a pseudo-emulsion,

**20.** The composition of claim 19, **characterized in that** said composition is in the form of a water/silicone oil emulsion or in the form of a multiple water/oil/water emulsion.

**21.** The use of a combination of 0.01 to 0.2% by weight of the total composition of at least one derivative of methylated silanol and 0.05 to 1% by weight of the total composition of at least one derivative of hydrolysed plant protein which is hydrolysed barley protein, hydrolysed wheat protein or hydrolysed oat protein for the preparation of a medicinal product as claimed in any one of claims 1 to 20 for the treatment of symptoms of skin ageing or for the treatment of the consequences of exposure to atmospheric pollution.

**Patentansprüche**

1.  Dermatologische und/oder kosmetische Zusammensetzung zur Behandlung der Symptome der Hautalterung, umfassend eine Verbindung von 0,01 bis 0,2 Gew.-% der Gesamtzusammensetzung von mindestens einem Derivat von methyliertem Silanol und von 0,05 bis 1 Gew.-% der Gesamtzusammensetzung von mindestens einem Derivat von hydrolysiertem Pflanzenprotein, das ein Protein von hydrolysierter Gerste, ein Protein von hydrolysiertem Weizen oder ein Protein von hydrolysiertem Hafer ist.

2.  Zusammensetzung nach Anspruch 1, bei der das Derivat von methyliertem Silanol das Methylsilanolmannuronat ist.

3.  Zusammensetzung nach Anspruch 1, bei der das Derivat von hydrolysiertem Pflanzenprotein ein Proteinextrakt von hydrolysiertem Weizen ist.

4.  Zusammensetzung nach einem der Ansprüche 1 bis 3, die ferner Vitamin C und/oder ein oder mehrere seiner Derivate enthält.

5.  Zusammensetzung nach Anspruch 4, bei der das Derivat von Vitamin C das Magnesiumascorbylphosphat ist.

6.  Zusammensetzung nach einem der Ansprüche 1 bis 5, die ferner ein oder mehrere aus einer pflanzlichen Quelle extrahierte Öle und/oder Phytinsäure enthält.

7.  Zusammensetzung nach Anspruch 6, bei der ein oder mehrere Öle aus Helianthus annuus und/oder Hedera helix extrahiert werden und die Phytinsäure aus Reiskleie extrahiert wird.

8.  Zusammensetzung nach einem der Ansprüche 1 bis 7, die ferner ein Oligopeptid oder ein Derivat desselben enthält.

9.  Zusammensetzung nach Anspruch 8, bei der das Derivat des Oligopeptids ein Palmitoyloligopeptid ist, bei dem das Oligopeptid aus Motiven von Glycin, Histidin und Lysin oder aus Motiven von Arginin, Glycin, Asparginsäure und Serin zusammengesetzt ist; das Oligopeptid ein Polypeptid ist, das aus Motiven von Arginin und Lysin zusammengesetzt ist, oder das Oligopeptid ein Polypeptid ist, das von Saccharomyces stammt.

10. Zusammensetzung nach einem der Ansprüche 1 bis 9, die ferner Vitamin E und/oder ein oder mehrere seiner Derivate enthält.

11. Zusammensetzung nach Anspruch 10, bei der das Vitamin E oder sein Derivat das DL-Tocopherol oder das Tocopherylacetat ist.

12. Zusammensetzung nach einem der Ansprüche 1 bis 11, die ferner Vitamin A und/oder ein oder mehrere seiner Derivate enthält.

13. Zusammensetzung nach Anspruch 12, bei der das Vitamin A oder sein Derivat Retinol, Retinylacetat oder Retinylpalmitat ist.

14. Zusammensetzung nach Anspruch 4 oder 5, bei der das Vitamin C und/oder seine Derivate in einer Menge von 0,25 bis 30 Gew.-% der Zusammensetzung vorhanden sind.

15. Zusammensetzung nach Anspruch 6 oder 7, bei der jedes pflanzliche Öl oder jedes der mehreren pflanzlichen Öle und/oder die Phytinsäure in einer Menge von ungefähr 0,005 bis 0,05% der Zusammensetzung vorhanden ist.

16. Zusammensetzung nach Anspruch 7, bei der das aus Helianthus annuus extrahierte Öl in einer Menge von ungefähr 0,01 bis 0,04% der Zusammensetzung vorhanden ist, das aus Hedera helix extrahierte Öl in einer Menge von ungefähr 0,01 bis 0,04% der Zusammensetzung vorhanden ist, und die Phytinsäure gegebenenfalls in einer Menge von ungefähr 0,01 bis 0,04% der Zusammensetzung vorhanden ist.

**17.** Zusammensetzung nach Anspruch 10 oder 11, bei der das Vitamin E und/oder seine Derivate in einer Menge von ungefähr 0,05 bis 2 Gew.-% der Zusammensetzung vorhanden sind.

**18.** Zusammensetzung nach Anspruch 12 oder 13, bei der das Vitamin A und/oder seine Derivate in einer Menge von ungefähr 0,02 bis 1 Gew.-% der Zusammensetzung vorhanden sind.

**19.** Zusammensetzung nach einem der Ansprüche 1 bis 18, **dadurch gekennzeichnet, daß** die Zusammensetzung in Form einer Wasser-/Ölemulsion, einer silikonierten Wasser-/Ölemulsion, einer Öl-/Wasseremulsion, einer mehrfachen Wasser-/Öl-/Wasseremulsion oder einer Pseudoemulsion vorhanden ist.

**20.** Zusammensetzung nach Anspruch 19, **dadurch gekennzeichnet, daß** die Zusammensetzung in Form einer silikonierten Wasser-/Ölemulsion oder in Form einer mehrfachen Wasser-/Öl-/Wasseremulsion vorhanden ist.

**21.** Verwendung einer Verbindung von 0,01 bis 0,2 Gew.-% der Gesamtzusammensetzung von mindestens einem Derivat von methyliertem Silanol und von 0,05 bis 1 Gew.-% der Gesamtzusammensetzung von mindestens einem Derivat von hydrolysiertem Pflanzenprotein, das ein Protein von hydrolysierter Gerste, ein Protein von hydrolysiertem Weizen oder ein Protein von hydrolysiertem Hafer ist, für die Herstellung eines Heilproduktes nach einem der Ansprüche 1 bis 20 zur Behandlung der Symptome der Hautalterung und zur Behandlung der Folgen der Luftverschmutzung.

**Revendications**

**1.** Composition dermatologique et/ou cosmétique pour le traitement des symptômes du vieillissement de la peau, comprenant une association de 0,01 à 0,2% en poids de la composition totale d'au moins un dérivé de silanol méthylé et de 0,05 à 1% en poids de la composition totale d'au moins un dérivé de protéine végétale hydrolysée, qui est une protéine d'orge hydrolysée, une protéine de blé hydrolysée ou une protéine d'avoine hydrolysée.

**2.** Composition selon la revendication 1, dans laquelle le dérivé de silanol méthylé est le mannuronate de méthylsilanol.

**3.** Composition selon la revendication 1, dans laquelle le dérivé de protéine végétale hydrolysée est un extrait de protéine de blé hydrolysée.

**4.** Composition selon l'une quelconque des revendications 1 à 3, contenant en outre de la vitamine C et/ou un ou plusieurs de ses dérivés.

**5.** Composition selon la revendication 4, dans laquelle le dérivé de la vitamine C est l'ascorbylphosphate de magnésium.

**6.** Composition selon l'une quelconque des revendications 1 à 5, contenant en outre une ou plusieurs huiles extraites d'une source végétale et/ou de l'acide phytique.

**7.** Composition selon la revendication 6, dans laquelle l'une ou plusieurs huiles sont extraites à partir d'Helianthus annuus et/ou d'Hedera helix et l'acide phytique est extrait à partir du son de riz.

**8.** Composition selon l'une quelconque des revendications 1 à 7, contenant en outre un oligopeptide ou une dérivé de celui-ci.

**9.** Composition selon la revendication 8, dans laquelle le dérivé de l'oligopeptide est un oligopeptide de palmitoyle dans lequel l'oligopeptide est composé de motifs de glycine, d'histidine et de lysine, ou de motifs d'arginine, de glycine, d'acide aspartique et de sérine ; l'oligopeptide est un polypeptide composé de motifs d'arginine et de lysine, ou l'oligopeptide est un polypeptide issu de Saccharomyces.

**10.** Composition selon l'une quelconque des revendications 1 à 9, contenant en outre de la vitamine E et/ou un ou plusieurs de ses dérivés.

**11.** Composition selon la revendication 10, dans laquelle la vitamine E ou son dérivé est le DL-tocophérol ou l'acétate de tocophéryle.

**12.** Composition selon l'une quelconque des revendications 1 à 11, contenant en outre de la vitamine A et/ou un ou plusieurs de ses dérivés.

**13.** Composition selon la revendication 12, dans laquelle la vitamine A ou son dérivé est le rétinol, l'acétate de rétinyle ou le palmitate de rétinyle.

**14.** Composition selon la revendication 4 ou la revendication 5, dans laquelle la vitamine C et/ou ses dérivés sont présents en une quantité allant de 0,25 à 30% en poids de la composition.

**15.** Composition selon la revendication 6 ou la revendication 7, dans laquelle chacune de l'une ou plusieurs huiles végétales et/ou l'acide phytique est présent(e) en une quantité allant d'environ 0,005 à 0,05% de la composition.

**16.** Composition selon la revendication 7, dans laquelle l'huile extraite à partir d'Helianthus annuus est présente en une quantité allant d'environ 0,01 à 0,04% de la composition, l'huile extraite à partir d'Hedera hélix est présente en une quantité allant d'environ 0,01 à 0,04% de la composition, et l'acide phytique (le cas échéant) est présent en une quantité allant d'environ 0,01 à 0,04% de la composition.

**17.** Composition selon la revendication 10 ou la revendication 11, dans laquelle la vitamine E et/ou ses dérivés sont présents en une quantité allant d'environ 0,05 à 2% en poids de la composition.

**18.** Composition selon la revendication 12 ou la revendication 13, dans laquelle la vitamine A et/ou ses dérivés sont présents en une quantité allant d'environ 0,02 à 1% en poids de la composition.

**19.** Composition selon l'une quelconque des revendications 1 à 18, **caractérisée en ce que** ladite composition est sous la forme d'une émulsion eau/huile, d'une émulsion eau/huile siliconée, d'une émulsion huile/eau, d'une émulsion multiple eau/huile/eau ou d'une pseudo-émulsion.

**20.** Composition selon la revendication 19, **caractérisée en ce que** ladite composition est sous la forme d'une émulsion eau/huile siliconée, ou sous la forme d'une émulsion multiple eau/huile/eau.

**21.** Utilisation d'une association de 0,01 à 0,2% en poids de la composition totale d'au moins un dérivé de silanol méthylé, et de 0,05 à 1% en poids de la composition totale d'au moins un dérivé de protéine végétale hydrolysée, qui est une protéine d'orge hydrolysée, une protéine de blé hydrolysée ou une protéine d'avoine hydrolysée, pour la préparation d'un produit médicinal selon l'une quelconque des revendications 1 à 20 pour le traitement des symptômes du vieillissement de la peau ou pour le traitement des conséquences d'une exposition à la pollution atmosphérique.

**Fig. 1**

**Fig. 2**

**Fig. 3**

**Fig. 4**

**Fig. 5**